# EUROPÄISCHE PATENTSCHRIFT

(11) **EP 0 934 750 B1**
(45) Veröffentlichungstag und Bekanntmachung des Hinweises auf die Patenterteilung: **20.07.2005**
(21) Anmeldenummer: 99101438.2
(22) Anmeldetag: 27.01.1999
(51) Int. Cl.: A61L 27/00, A61F 2/08

(54) **Biohybrider Gelenkflächenersatz**
Bio-hybrid Joint surface substitute
Remplacement bio-hybride de surface d'articulation

(30) Priorität: 30.01.1998 DE 19803673
(43) Veröffentlichungstag der Anmeldung: 11.08.1999
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Meenen, Norbert M. Dr., 22587 Hamburg (DE); Dauner, Martin, 73732 Esslingen (DE); Planck, Heinrich Dr., 72622 Nürtingen (DE)
(74) Vertreter: Patentanwälte Ruff, Wilhelm, Beier, Dauster & Partner

(56) Entgegenhaltungen:
- WO-A-90/12603
- WO-A-94/20151
- WO-A-95/31157
- WO-A-97/14783
- US-A- 4 457 028
- US-A- 5 674 292

## Beschreibung

Die Erfindung betrifft einen biohybriden Gelenkflächenersatz.

Zur Behandlung von geschädigten Gelenkknorpeln, etwa durch Mißbildungen, Verletzungen oder degenerative Erkrankungen, konzentrieren sich Entwicklungsarbeiten auf die Herstellung von Ersatzknorpeln, die dem Patienten implantiert werden können.

Es sind aus jüngster Zeit Verfahren zum Herstellen von Implantaten aus Zellkulturen bekannt wie sie beispielsweise in der internationalen Patentanmeldung WO 94/20151 von Sittinger beschrieben werden. Hierbei werden in den Hohlräumen einer dreidimensionalen Trägerstruktur Knorpelzellen in vitro hergestellt.

In WO 90/12603 von Vacanti ist ein System zur Neomorphogenese von Knorpelzellen aus einer Zellkultur in vivo offenbart.

Aus der WO 97/14783 ist ein Verfahren zur Herstellung einer Zusammensetzung zur Reparatur von Knochendefekten bekannt, welche aus einer Matrix aus Hydroxyapatit, synthetischen Polymeren oder natürlichen Polymeren bestehen kann und wobei die Zusammensetzung insgesamt bioresorbierbar sein kann. Dabei werden Zellen auf der bioverträglichen Matrix zur Ausbildung von neuen Knochen kultiviert.

Die aus dem Stand der Technik bekannten Knorpelimplantate weisen jedoch in der Praxis eine Reihe von Nachteilen auf.

Für einen vollwertigen Gelenkflächenersatz ist eine zuverlässige Fixierung im subchondralen Knochen wichtig. Dazu muß das Implantat durch aufwendige Nahttechniken oder Befestigungsklammern gehalten werden.

Ein wesentliches Problem bei der Therapie von pathologischen Veränderungen von Gelenkoberflächen ist das Eindringen von Gelenkflussigkeit (Synovialflüssigkeit), deren Bestandteile wie Hyaluronsäure die Regenerierung und das Einwachsen beeinträchtigen. Die Gelenksflüssigkeit, die das eingesetzte Implantat umspült, verhindert ein Festwachsen der Knorpelstruktur an den Defektgrenzen und am Knochen.

Die bisher bekannten Knorpelersatzstoffe zur Behandlung von Gelenkdefekten können die für eine erfolgreiche und dauerhafte Heilung geforderten Kriterien wie sichere und einfache Befestigung am Knochen, rasches Einwachsen, physiologische Wiederherstellung der Gelenkstruktur und dauerhafte Funktionalität nicht erfüllen.

Es stellt sich daher die Aufgabe, einen Gelenkflächenersatz zur Verfügung zu stellen, der aus Knorpelzellen in einer dreidimensionalen Struktur gebildet ist, der unter üblichen biomedizinischen Bedingungen und mit vorhandenen Einrichtungen einfach und sicher herzustellen ist.

Diese Aufgabe wird gelöst durch einen biohybriden Gelenkflächenersatz in Form eines dreidimensionalen porösen Trägers, in dem sich Knorpelzellen in vitro und/oder in vivo zu einem dreidimensionalen Zellverband kultivieren lassen, und der nach dem Zellwachstum, und gegebenenfalls nach einer Gewebeentwicklung, im Bereich einer defekten Gelenkfläche auf den freigelegten Knochen aufsetzbar ist, dadurch gekennzeichnet, daß der Träger an der zur Anlage an den Knochen bestimmten Seite Mittel zur Förderung der knöchernen Integration aufweist, und dass mindestens eine resorbierbare Versiegelungsschicht in Form einer Folie, Membran und/oder Dichtmasse vorgesehen ist, welche vor dem Zutritt von Synovialflüssigkeit in die Berührungsfläche zwischen dem Integrationsvermittler und Knochen schützt. Die mindestens eine Versiegelungsschicht verhindert, dass in die Fläche zwischen dem Integrationsvermittler und dem Knochen Synovialflüssigkeit eindringt. Mögliche Versiegelungsmittel sind Folien, Membranen und/oder Dichtmassen, je nach Art der Applikation.

Zur Herstellung von Ersatzknorpelzellen kann zunächst eine Zellvermehrung von proliferierenden Knorpelzellen (dedifferenzierten sogenannten fibroblastlike-cells) vorgenommen werden. Dies kann beispielsweise als flächiges Zellwachstum in einem Zellkulturgefäß erfolgen. Nach einer gewissen Vermehrung der Zellen werden die Zellen dann wieder spezifisch gemacht (differenziert) und für das weitere dreidimensionale Wachstum in eine geeignete Matrix eingebracht. Insbesondere können die Knorpelzellen in die Matrix eingespritzt oder mit leichtem Unterdruck in die Matrix eingesaugt oder einzentrifugiert werden. Bevorzugt kann eine solche Matrix- oder Trägerstruktur wenige Wochen vor der geplanten Implantation mit körpereigenen Zellen des Patienten versehen werden.

Insbesondere kann der poröse Träger aus unter physiologischen Bedingungen im Körper resorbierbarem Material bestehen. Dieses kann natürlichen oder synthetischen Ursprungs sein. Mit Vorteil kann der poröse Träger in Form eines Vlieses, Schaums, Schwamms, Sintermaterials oder einer textilen Struktur vorliegen.

Der poröse Träger aus resorbierbarem Material kann sich dadurch auszeichnen, daß er eine Porosität von mehr als 65 %, insbesondere mehr als 75 % aufweist. Vorzugsweise ist der Träger ein hochporöser Träger. Er kann eine Porosität von mehr als 90 %, bevorzugt mehr als 95 % aufweisen.

Der poröse Träger aus resorbierbarem Material kann aus hydrolytisch abbaubaren Polymeren, insbesondere hydrolysierbaren Polymeren der a- und β-Hydroxycarbonsäuren gebildet sein. Vorzugsweise besteht er aus einem oder mehreren resorbierbaren synthetischen Polymeren auf Basis der Polyester der Hydroxycarbonsäuren. Als Beispiele für biologisch abbaubaren Materialien sind Polymere auf Basis von Polyglykolsäure, Polymere der Glykolsäure (PGA), Polylactide, Polymere von L-Lactid (P-L-LA), D-Lactid (P-D-LA), DL-Lactid (P-DL-LA), Polydioxanon, Polycaprolacton, Copolymere und Terpolymere aus den genannten Monomeren und/oder Mischungen aus den genannten Polymeren zu nennen. Mit Vorteil können Copolymere von Glykolsäure und Milchsäure im Verhältnis 99:1 bis 1:99, insbesondere im Verhältnis 89:11 bis 70:30 oder von 30:70 bis 11:89 verwendet werden. Außerdem können vorteilhaft Copolymere von L-Lactid und DL-Lactid im Verhältnis 99:1 bis 1:99, insbesondere im Verhältnis 99:1 bis 70:30 verwendet werden. Die erfindungsgemäß zu verwendenden Copolymere, Terpolymere und/oder Mischungen aus den oben genannten Monomeren bzw. Polymeren können zur Verbesserung ihrer Eigenschaften mit weichmachenden Substanzen, insbesondere Caprolacton, Trimethlyencarbonat (TMC), Triethylcitrat (TEC) und/oder Acetylbutylcitrat (AtBC) versehen sein.

Unter Berücksichtigung der entsprechenden Vorkehrungen zum Ausschluß immunologischer Probleme oder infektiöser Krankheiten kann der poröse Träger aus natürlichen resorbierbaren Substanzen, mit Vorteil aus Collagen oder aus Hyaluronsäure und ihrer Derivate, hergestellt sein.

Der poröse Träger kann aus einem Schaum, Schwamm, Sintermaterial oder aus einer textilen Struktur gebildet sein, wobei Spinnvliese oder Spinnfaservliese bevorzugt sind.

Im Falle von porösen Trägern aus Fasermaterial, wie Vliesen oder anderen textilen Flachengebilden, kann der Faserdurchmesser vorteilhaft 1 bis 50 µm, insbesondere 5 bis 15 µm betragen.

Zur besseren Steuerung der Knorpelbildung und zur Beeinflußung der Degradationszeit können poröse Träger aus einer Kombination von Fasermaterialien bestehen. Dies kann durch den Einsatz von Fasermischungen, oder durch Mischfasern, Bikonstituentenfasern oder mit Bikomponenten-Fasern geschehen. Eine gesteuerte Degradation kann hierbei mit Vorteil durch die Verwendung von Bikomponenten-Fasern des Typs Kern/Mantel oder des Insel-Typs erreicht werden.

Schichtförmige Träger (z. B. Vliese) können eine Dicke von 1 bis 5 mm aufweisen. Beispielsweise können bei Implantaten zur Anwendung am Knie eines Menschen Schichtdicken von 1 bis 3 mm verwendet werden. Für Anwendungen an der Hüfte können wegen der höheren Beanspruchung größere Dicken zweckmäßig sein.

Der erfindungsgemäße Gelenkflächenersatz kann sich dadurch auszeichnen, daß der Integrationsvermittler fest an den porösen Träger gebunden ist. Mit Vorteil kann der Integrationsvermittler in eine Versiegelungsschicht partiell eingebettet sein. Als Beispiel für erfindungsgemäß geeignete Versiegelungsmittel sind Folien oder Membranen aus den im Zusammenhang mit dem porösen Träger aufgeführten resorbierbaren Polymeren zu nennen.

Der Integrationsvermittler kann in feinteiliger Form vorliegen, wobei die mittlere Teilchengröße vorzugsweise zwischen 10 und 500 µm, insbesondere 50 bis 100 µm beträgt.

Der Integrationsvermittler kann erfindungsgemäß in einer Menge vorhanden sein, die die Oberfläche der dem Knochen zugewandten Seite mindestens zu 30 % bis 100 %, vorzugsweise zu 95 % bis 100 % bedeckt.

Mit Vorteil kann der Integrationsvermittler von einer anorganischen Knochengrundsubstanz gebildet werden, insbesondere aus einer solchen bestehen.

Als Beispiele für gemäß der Erfindung bevorzugte Integrationsvermittler sind Hydroxylapatitkeramik, Tricalciumphosphatkeramik, Misch-Keramiken und/oder Calciumcarbonat zu nennen. Auch amorphe Formen der anorganischen Knochengrundsubstanzen kommen in Frage.

Der Integrationsvermittler kann insbesondere eine poröse Struktur besitzen, wobei die Porosität 30 bis 80 Vol-% oder mehr beträgt und die Porengröße im Bereich von 10 bis 150 um liegt. Der Integrationsvermittler kann auch aus einer zusammenhängenden hochporösen Platte bestehen.

In einer Ausführungsform der Erfindung kann der Integrationsvermittler biologischen Ursprungs sein, insbesondere aus hochtemperaturbehandeltem Knochen bestehen. Eine solche Hochtemperaturbehandlung kann insbesondere bei Temperaturen über 1000 °C erfolgen. Ein auf diese Weise behandeltes Knochenmaterial ist eine biologische Hydroxylapatitkeramik.

In einer anderen Ausführungsform der Erfindung kann der Integrationsvermittler aus Keramik vollsynthetischen Ursprungs gebildet sein. Zur Herstellung einer solchen Keramik können den Fachleuten aus dem Stand der Technik bekannte Herstellungsverfahren verwendet werden.

Zur Herstellung eines Gelenkknorpelbiohybrids, einem kultivierten Gelenkknorpel in geeigneter dreidimensionaler Raumstruktur gemäß der vorliegenden Erfindung ist eine Basis erforderlich, die mit dem Knochen eine dauerhafte und kraftschlüssige Verbindung eingeht. Insbesondere kann ein Granulat von Hydroxylapatitkeramik verwendet werden, das bioaktiv und osteokonduktiv eine schnelle substanzielle Integration durch Aufwachsen von interstizieller Matrix auf das synthetische poröse Material fördert.

Erfindungsgemäß kann das Granulat in eine resorbierbare Polymerfolie eingearbeitet werden. Der erfindungsgemäße Gelenkflächenersatz kann dadurch gekennzeichnet sein, daß die Teilchen des Integrationsvermittlers teilweise in eine Schicht eingebettet sind und ein Teil ihrer Oberfläche frei liegt. Auf diese Weise ist nach Degradation der Polymerbestandteile des biohybriden Implantats der Gelenkknorpel mit seiner Matrix an einer keramischen Basis fixiert, die wiederum zuverlässig Halt im subchondralen Knochen findet.

Erfindungsgemäß können die Hydroxylapatitkeramikpartikel in einem Monolayer in einer Polymerfolie vorgesehen sein. Als Beispiel für erfindungsgemäß geeignete Polymerfolien sind Folien oder Membranen aus den im Zusammenhang mit dem porösen Träger aufgeführten resorbierbaren Polymeren zu nennen.

Bevorzugt kann eine Polymerfolie verwendet werden, die eine Dicke von ungefähr 100 µm bis 300 µm aufweist. Mit eingebetteten Partikeln kann die Polymerfolie insbesondere eine Dicke von bis zu 600 µm aufweisen. Die Partikelgröße im Monolayer kann 10 bis 500 µm, insbesondere 50 bis 100 µm betragen.

Der biohybride Gelenkflächenersatz sollte in idealer Weise an die entsprechende Oberfläche des zu ersetzenden Gelenkflächenbereichs angepasst sein. Der dreidimensionale Träger und/oder der Integrationsvermittler können gemäß der vorliegenden Erfindung konkav, konvex oder in einer anderen gekrümmten Raumform angepasst an den natürlichen, zu ersetzenden Gelenkknorpel vorliegen. Dabei kann die dem Knochen zugewandte Seite des Integrationsvermittlers mit Vorteil eben sein, um die Implantation zu erleichtern.

Die geeignete Raumform lässt sich nicht-invasiv durch bildgebende Verfahren, wie der Röntgenstrahlen-Computertomographie oder der MRT, bestimmen und durch ein Rapid-Prototyping-Verfahren als Modell oder Matrize nachbilden.

Der Gelenkflächenersatz gemäß der vorliegenden Erfindung zeichnet sich vorzugsweise dadurch aus, daß mindestens eine Versiegelungsschicht vorgesehen ist, die sowohl die Berührungsfläche zwischen Integrationsvermittler und Knochen als auch zwischen Implantat und Defektgrenzen am Knorpel im implantierten Zustand vor Zutritt von Synovialflüssigkeit schützt. Die mindestens eine Versiegelungsschicht verhindert, dass in die Fläche zwischen dem Implantat in Form des porösen Trägers und den Grenzen des Defektes am Knorpel sowie in die Fläche zwischen dem Integrationsvermittler und dem Knochen Synovialflüssigkeit eindringt. Die möglichen Versiegelungsmittel sind Folien, Membranen und/oder Dichtmassen, je nach Art der Applikation, wie z. B. an der Oberfläche des Implantats, auf der Defektgrenze am Knorpel und/oder auf der Grenzfläche zwischen porösem Träger und Integrationsvermittler.

Erfindungsgemäß kann die synthetische Stützstruktur zur Defektgrenze hin in der Weise begrenzt sein, daß sie für Synovialflüssigkeit und Zellen undurchlässig ist. Auf diese Weise können Stoffe, die eine zuverlässige Verankerung des Implantats im Knochen beeinträchtigen, ferngehalten werden.

Erfindungsgemäß ist beim Gelenkflächenersatz zwischen Integrationsvermittler und Knochen und vorzugsweise zwischen Implantat und Defektgrenzen am Knorpel im implantierten Zustand zum Schutz der Berührungsfläche vor Zutritt von Flüssigkeit eine Versiegelungsschicht vorgesehen. Als Beispiel für erfindungsgemäß geeignete Versiegelungsmittel sind Folien oder Membranen aus den im Zusammenhang mit dem porösen Träger aufgeführten resorbierbaren Polymeren zu nennen.

In einer weiteren Ausführungsform der Erfindung kann beim Gelenkflächenersatz zum Schutz der Beruhrungsfläche zwischen Implantat und Defektgrenzen am Knorpel und zwischen Integrationsvermittler und Knochen im implantierten Zustand vor Zutritt von Flüssigkeit eine semipermeable Membran vorgesehen sein. Als Beispiel für erfindungsgemäß geeignete semipermeable Membranen sind Folien oder Membranen aus den im Zusammenhang mit dem porösen Träger aufgeführten resorbierbaren Polymeren zu nennen.

Der Cut-off einer solchen Membran kann insbesondere bei weniger als 60000 Dalton liegen. Auf diese Weise können größere Moleküle, wie beispielsweise Proteine, und Zellen am Durchtritt durch die Membran gehindert werden.

In noch einer Ausführungsform der Erfindung kann beim Gelenkflächenersatz zum Schutz der Berührungsfläche zwischen Implantat und Defektgrenze am Knorpel und zwischen Integrationsvermittler und Knochen im implantierten Zustand vor Zutritt von Flüssigkeit eine mikroporöse Membran vorgesehen sein, deren Porengröße bevorzugt unter 1 µm beträgt. Als Beispiel für erfindungsgemäß geeignete mikroporöse Membranen sind Mikrovliesstoffe oder Folien oder Membranen aus den im Zusammenhang mit dem porösen Träger aufgeführten resorbierbaren Polymeren zu nennen.

Mit Vorteil kann eine schützende Schicht vorgesehen sein, die vor allem für Zellen und Synovialflüssigkeit eine Barriere bildet, aber in einer Weise permeabel ist, um Stoffwechsel zu ermöglichen. Ein möglichst ungehinderter Stoffwechsel ist für ein schnelles Anwachsen und eine substantielle Integration des Implantats von Bedeutung.

Als Beispiele für Polymermaterialien, aus denen eine Trennschicht, Versiegelungsschicht, Polymerfolie, geschlossene Schicht, Schutzschicht, Membranen usw. hergestellt sein können, sind Polylactide, Polymere von L-Lactid (P-L-LA), D-Lactid (P-D-LA), DL-Lactid (P-DL-LA), Polydioxanon, Polycaprolacton, Copolymere und Terpolymere aus den genannten Monomeren und/oder Mischungen aus den genannten Polymeren sowie Mischungen der genannten Polymere mit Polyglykolsäure zu nennen. Mit Vorteil können Copolymere von Glykolsäure und Milchsäure im Verhältnis 90:10 bis 1:99, insbesondere im Verhältnis 30:70 bis 11:89 verwendet werden. Außerdem können vorteilhaft Copolymere von L-Lactid und DL-Lactid im Verhältnis 99:1 bis 1:99, insbesondere im Verhältnis 99:1 bis 70:30 verwendet werden. Die erfindungsgemäß zu verwendenden Copolymere, Terpolymere und/oder Mischungen aus den oben genannten Monomeren bzw. Polymeren können zur Verbesserung ihrer Eigenschaften mit weichmachenden Substanzen, insbesondere Caprolacton, Trimethlyencarbonat (TMC), Triethylcitrat (TEC) und/oder Acetylbutylcitrat (AtBC) versehen sein.

Unter Berücksichtigung der entsprechenden Vorkehrungen zum Ausschluß immunologischer Probleme oder infektiöser Krankheiten können Trennschicht, Versiegelungsschicht, Polymerfolie, geschlossene Schicht, Schutzschicht, Membranen usw. aus natürlichen resorbierbaren Substanzen, mit Vorteil aus Collagen oder aus Hyaluronsäure und ihrer Derivate hergestellt sein.

Mit Vorteil können für die erfindungsgemäßen Trennschichten usw. zu verwendende Polymere aus Lösung verarbeitbar sein. In einer anderen Ausführungsform der Erfindung können die für die erfindungsgemäße Trennschicht zu verwendenden Polymere thermoplastisch verarbeitbar sein.

In einer weiteren Ausführungsform der Erfindung können die für die erfindungsgemäße Trennschicht zu verwendenden Polymere vernetzend verarbeitbar sein.

Der erfindungsgemäße Gelenkflächenersatz kann sich vorteilhaft dadurch auszeichnen, daß die geschlossene Schicht zumindest während der anfänglichen Einwachszeit nach der Implantation im wesentlichen undurchlässig für Flüssigkeiten ist.

In einer weiteren Ausführungsform kann der erfindungsgemäße Gelenkflächenersatz dadurch gekennzeichnet sein, daß ihm eine Deckfolie zugeordnet ist, die zum Abdecken der dem Integrationsvermittler abgewandten Oberseite des Trägers bestimmt ist. Mit Vorteil kann die Deckfolie auf dem Träger befestigt, insbesondere adhäsiv/kohäsiv, physisch zugeordnet sein. Als Materialien für die erfindungsgemäße Deckfolie können Polymermaterialien verwendet werden, wie sie oben für die Trennschicht vorgeschlagen wurden. Die Deckfolie kann insbesondere aus resorbierbarem Material bestehen. Ferner kann die Deckfolie vorzugsweise aus einer porösen Membran, die ein Eindringen von Synovialflüssigkeit verhindert, bestehen.

Mit Vorteil kann die Deckfolie größer sein als die Fläche der Trägeroberseite und überstehende Ränder aufweisen, die zum Überdecken der Trennlinie zwischen Gelenkflächenersatz und nativer Gelenkknorpeloberfläche vorgesehen ist.

Zur Befestigung von Deckfolie auf dem porösen Träger kann mit Vorteil chirurgisches Nahtmaterial aus bioresorbierbarem Material verwendet werden. Die Degradationszeit des Nahtmaterials kann im Bereich der Degradationszeit der resorbierbaren Deckfolie liegen. Eine sichere Befestigung kann sichergestellt werden, wenn die Resorptionsdauer des Nahtmaterials insbesondere länger ist als die der Deckfolie. Geeignete Resorptionszeiten für die Folie können beispielsweise im Bereich von 1 bis 4 Monaten liegen. Besonders bevorzugt sind erfindungsgemäß Nahtmaterialien auf Basis von Polyglykolsäure. Weitere Befestigungsmittel sind Gewebekleber, resorbierbare Stifte oder Klammern oder Collagen.

Alternativ oder ergänzend zur Deckfolie kann dem erfindungsgemäßen Gelenkflächenersatz eine resorbierbare Dichtmasse zugeordnet sein, die zwischen porösen Träger und Defektgrenze eingebracht oder auf die oberflächige Trennlinie von Implantat zu Gelenkfläche aufgebracht, ein Eindringen der makromolekularen Bestandteile der Synovialflüssigkeit verhindert. Die resorbierbare Dichtmasse kann z. B. aus Gewebekleber oder Collagen bestehen.

Mit Vorteil kann der Gelenkflächenersatz gemäß der Erfindung sich dadurch auszeichnen, dass der poröse Träger und/oder der Integrationsvermittler eine gekrümmte Raumform angepasst an die natürliche Gelenkform aufweisen, wobei die dem Knochen zugewandte Seite des Integrationsvermittler bevorzugt eben ist.

Zur Verwendung als Implantat kann der erfindungsgemäße Gelenkflächenersatz in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfaßt die Behandlung mit ionisierender Strahlung wie beispielsweise Bestrahlung mit β-Strahlen oder γ-Strahlen, im Bereich von 0,1 Mrad bis 10 Mrad, insbesondere 0,8 Mrad bis 2,5 Mrad. In einer möglichen Ausführungsform der Erfindung kann eine mit Hilfe von Bestrahlen durchgeführte Sterilisation gleichzeitig zur Steuerung des Degradationsverhaltens des erfindungsgemäß hergestellten Gelenkflächenersatzes dienen.

Bei der Behandlung mit γ-Strahlen kann es zu einer Kettenspaltung beim resorbierbaren Polymermaterial kommen, was die Resorbierbarkeit im Körpermilieu verbessert, aber gleichzeitig keinen nennenswerten Festigkeitsverlust bedingt.

Zur Verbesserung der Hydrophilie des Polymerwerkstoffs können Modifizierungen der Polymeroberfläche vorgenommen werden. Mit Vorteil kann es bei einer Plasmabehandlung durch Reaktionen an der Oberfläche des Materials zu einer Modifizierung der funktionellen Gruppen kommen. Auf diese Weise kann auch eine Erhöhung der hydrophilen Eigenschaften erreicht werden. In bevorzugten Ausführungsformen kann eine solche Hydrophilierung und/oder Funktionalisierung der Oberfläche mit Niedertemperatur-Plasma vorgenommen werden. Mit Vorteil können die Modifizierungsreaktionen in Inertgasatmosphäre, Sauerstoff, CO2 und/oder SO2 ausgeführt werden. In einer weiteren Ausführungsform der Erfindung kann eine Plasmapfropfung mit Monomeren und/oder Oligomeren zur Polymeroberflächenmodifikation vorgenommen werden. Eine Oberflächenmodifikation kann die Besiedelung mit Zellen erleichtern und somit das Einwachsen des Implantats und die Regeneration des behandelten Gelenks günstig beeinflußen.

Zur Erleichterung der Regenerierung nach der Implantation kann das erfindungsgemäße Implantat mit Wachstumsfaktoren in wirksamer Konzentration versehen sein. Es sind sowohl Wachstumsfaktoren für Knorpelzellen wie Wachstumsfaktoren für Knochenzellen oder Kombinationen davon einsetzbar. Wachstumsfaktoren für Knochenzellen können gemäß der Erfindung bevorzugt in der Folie für den Integrationsvermittler eingelagert sein. Als Beispiele sind im Bereich der Physiologie und Medizin bekannte Wachstumsfaktoren wie zum Beispiel osteogenes Protein, BFGF (basic fibroblast growth factor), BMP (bone morphogenic protein), rh BMP (recombinant human bone morphogenic protein), TGF-β (transforming growth factor), IGF (insulin-like growth factor) und EGF (endothelial growth factor) zu nennen. Geeignete Konzentrationen an Wachstumsfaktoren können im Bereich von 0,1 bis 50 ng/ml liegen.

Während der Resorption der für den erfindungsgemäßen biohybriden Gelenkfächenersatz verwendeten biodegradierbaren Polymere kann es zu Schwankungen des pH-Werts kommen. Mit Vorteil können zur Abpufferung basische Salze in die Polymere und/oder die Poren des Implantats eingebracht werden. Als Beispiele für Puffersalze sind Calciumcarbonat, Natriumcarbonat, Natriumtartrat und/oder andere unter physiologischen Bedingungen geeignete Puffersysteme zu nennen. Die Pufferverbindungen können mit Vorteil in einer Konzentration und 0,1 bis 20 Gew.-%, bevorzugt 1 bis 10 Gew.-%, bezogen auf das Gewicht an resorbierbarem Polymer enthalten sein.

Mit dem erfindungsgemäßen Gelenkflächenersatz wird ein Implantat zur Wiederherstellung eines geschädigten Gelenkknorpels zur Verfügung gestellt, das die Züchtung eines funktionellen Knorpeltransplantates mit autologem Knorpel ermöglicht und eine sichere Verankerung im subchondralen Knochen gewährleistet. Zur Verbesserung der Verankerung des Implantats im Knochen kann der Defekt zuvor auf Maß gestanzt, gefräst oder geschnitten werden, so daß eine entsprechend angepaßte Implantatstruktur darin eingepreßt werden kann. Das Implantatstück kann aus einem zuvor großflächig hergestellten Knorpelersatz vorzugsweise zylindrisch vorgebildet sein. Dabei können mit Vorteil Durchmesser von 10 bis 15 mm vorgesehen sein.

Weitere Merkmale und Einzelheiten der Erfindung ergeben sich aus der nachfolgenden Beschreibung von bevorzugten Ausführungsformen anhand von Beispielen mit Bezugnahme zu den begleitenden Zeichnungen. Die Beispiele dienen zur Erläuterung bevorzugter Ausführungsformen, wobei die Erfindung in keiner Weise darauf beschränkt sein soll.

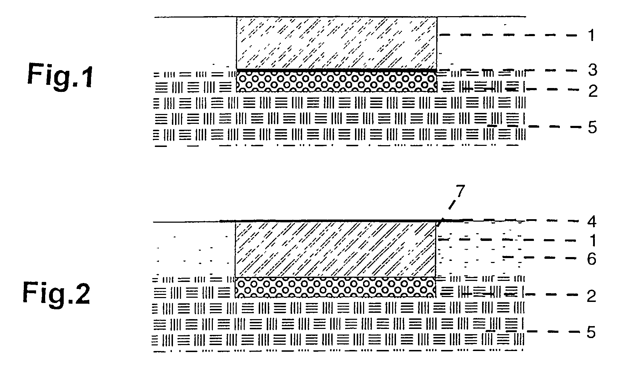

In den Zeichnungen zeigt Figur 1 einen erfindungsgemäßen Gelenkflächenersatz wie er in Beispiel 1 erläutert ist. Figur 2 zeigt einen erfindungsgemäßen Gelenkflächenersatz wie er in Beispiel 2 erläutert ist.

### Beispiel 1

Ein poröser Träger 1 wird als Vliesstruktur aus resorbierbaren Polymerfasern, hergestellt aus Copolymer von Glykolsäure und Milchsäure, gebildet. Der Träger 1 weist ein Porenvolumen von 95 % auf und ist fest mit einem Integrationsvermittler 2 verbunden. Als Integrationsvermittler 2 wird granuläre Hydroxylapatitkeramik mit einer mittleren Korngröße von 0,3 mm verwendet. Zur Verbindung von porösem Träger 1 und Integrationsvermittler 2 dient eine Versiegelungsschicht 3 aus einer resorbierbaren Polymermembran, hergestellt aus dem gleichen Copolymer von Glykolsäure und Milchsäure. Hauptaufgabe der Polymermembran ist, die Hydroxylapatitkeramik und den nach der Implantation darunter befindlichen subchondralen Knochen 5 vor der integrationshemmenden Synovialflüssigkeit zu schützen.

### Beispiel 2

Ein poröser Träger 1 entsprechend wie in Beispiel 1 ist mit einer Deckschicht 4 aus einer resorbierbaren Polymermembran, hergestellt aus einem Polyglykolid-trimethylencarbonat-copolymer versehen. Die Polymermembran ist durch eine zentrale Knopfnaht, ausgeführt mit resorbierbarem Nahtmaterial aus Polyglykolsäure, fest mit dem porösen Träger 1 verbunden. Die Membran überdeckt die Passfuge 7 des eingepreßten Implantates, und ist geeignet, nach entsprechender Fixierung auf dem umliegenden Knorpel 6, das gesamte Implantat und das anschließende Körpergewebe vor dem Eindringen der integrationshemmenden Synovialflüssigkeit zu schützen. Der Integrationsvermittler 2 besteht aus einer Platte aus poröser Hydroxylapatitkeramik, die bei entsprechender Vorbereitung des Implantatlagers eine besonders feste primäre Verankerung ermöglicht.

## Patentansprüche

1. Biohybrider Gelenkflächenersatz in Form eines dreidimensionalen porösen Trägers (1), in dem sich Knorpelzellen in vitro und/oder in vivo zu einem dreidimensionalen Zellverband kultivieren lassen, und der nach dem Zellwachstum, und gegebenenfalls nach einer Gewebeentwicklung, im Bereich einer defekten Gelenkfläche auf den freigelegten Knochen (5) aufsetzbar ist, **dadurch gekennzeichnet, dass** der Träger (1) an der zur Anlage an den Knochen (5) bestimmten Seite Mittel zur Förderung der knöchernen Integration (2) aufweist, und dass mindestens eine resorbierbare Versiegelungsschicht (3, 4) in Form einer Folie, Membran und/oder Dichtmasse vorgesehen ist, welche vor dem Zutritt von Synovialflüssigkeit in die Berührungsfläche zwischen dem Gelenkflachenersatz (1, 2) und Knochen (5) schützt.

2. Gelenkflächenersatz nach einem der vorhergehenden Ansprüche, **dadurch gekennzeichnet, dass** der poröse Träger (1) aus resorbierbarem Material besteht und bevorzugt aus mindestens einem resorbierbaren synthetischen Polymeren auf Basis der Polyester der α-Hydroxycarbonsäuren besteht.

3. Gelenkflächenersatz nach einem der vorhergehenden Ansprüche, **dadurch gekennzeichnet, dass** der poröse Träger (1) aus einem Schaum, Schwamm, Sintermaterial oder einer textilen Struktur, insbesondere einem Spinnvlies oder Spinnfaservlies gebildet wird.

4. Gelenkflächenersatz nach einem der vorhergehenden Ansprüche, **dadurch gekennzeichnet, dass** der Integrationsvermittler (2) fest an den porösen Träger (1) gebunden ist.

5. Gelenkflächenersatz nach einem der vorhergehenden Ansprüche, **dadurch gekennzeichnet, dass** der Integrationsvermittler (2) in feinteiliger Form vorliegt, wobei die mittlere Teilchengröße vorzugsweise zwischen 10 und 500 µm, insbesondere 50 bis 100 µm beträgt.

6. Gelenkflächenersatz nach einem der vorhergehenden Ansprüche, **dadurch gekennzeichnet, dass** der Integrationsvermittler (2) aus einer zusammenhängenden, hochporosen Platte besteht.

7. Gelenkflächenersatz nach einem der vorhergehenden Ansprüche, **dadurch gekennzeichnet, dass** der Integrationsvermittler (2) in einer Menge vorhanden ist, die die Oberfläche der dem Knochen (5) zugewandten Seite zu 30 % bis 100 %, vorzugsweise zu 95 % bis 100 % bedeckt.

8. Gelenkflächenersatz nach einem der vorhergehenden Ansprüche, **dadurch gekennzeichnet, dass** der Integrationsvermittler (2) von einer anorganischen Knochengrundsubstanz gebildet wird, vorzugsweise aus einer solchen besteht und insbesondere aus keramischen oder amorphen Hydroxylapatiten, Tricalciumphosphaten, Misch-Calciumphosphaten und/oder Calciumcarbonat besteht.

9. Gelenkflächenersatz nach einem der vorhergehenden Ansprüche, **dadurch gekennzeichnet, dass** der Integrationsvermittler (2) biologischen Ursprungs ist, insbesondere aus hochtemperaturbehandeltem Knochen besteht.

10. Gelenkflächenersatz nach einem der vorhergehenden Ansprüche, **dadurch gekennzeichnet, dass** der Integrationsvermittler (2) eine poröse Struktur besitzt, wobei die Porosität vorzugsweise 30 bis 80 Vol-% beträgt und die Porengröße bevorzugt im Bereich von 10 bis 150 µm liegt.

11. Gelenkflachenersatz nach einem der vorhergehenden Ansprüche, **dadurch gekennzeichnet, dass** die mindestens eine Versiegelungsschicht (3, 4) die Berührungsfläche zwischen Implantat (1, 2) und Defektgrenzen am Knorpel (6) und zwischen dem Integrationsvermittler (2) und Knochen (5) im implantierten Zustand vor Zutritt von Synovialflüssigkeit schützt.

12. Gelenkflächenersatz nach einem der vorhergehenden Ansprüche, **dadurch gekennzeichnet, dass** der dreidimensionale poröse Träger (1) an der Seite des Integrationsvermittlers (2) mit einer geschlossenen Schicht (3) versehen ist, insbesondere mit einer vorzugsweise resorbierbaren Folie oder Membran verbunden ist, in der die Teilchen des Vermittlers verankert sind.

13. Gelenkflächenersatz nach einem der vorhergehenden Ansprüche, **dadurch gekennzeichnet, dass** ihm eine vorzugsweise resorbierbare Deckfolie oder Membran (4) zugeordnet ist, die zum Abdecken der dem Integrationsvermittler (2) abgewandten Oberseite des Trägers (1) und insbesondere der Kontaktfuge (7) zwischen Implantat und nativer Gelenkoberfläche bestimmt ist.

14. Gelenkflächenersatz nach einem der vorhergehenden Ansprüche, **dadurch gekennzeichnet, dass** ihm eine resorbierbare Dichtmasse zugeordnet ist, die zwischen Implantat und Implantatlager eingebracht, oder auf die oberflächige Trennlinie von Implantat zu Gelenkfläche aufgebracht, ein Eindringen der makromolekularen Bestandteile der Synovialflüssigkeit verhindert.

15. Gelenkflächenersatz nach einem der vorhergehenden Ansprüche, **dadurch gekennzeichnet, dass** der poröse Träger (1) und/oder der Integrationsvermittler (2) eine gekrümmte Raumform angepasst an die natürliche Gelenkform aufweisen, wobei die dem Knochen (5) zugewandte Seite des Integrationsvermittlers (2) bevorzugt eben ist.

## Claims

1. Biohybrid articular surface replacement device in the form of a three-dimensional porous carrier (1) in which cartilage cells can be cultivated in vitro and/or in vivo to form a three-dimensional cell union and which, after cell growth and, as the case may be, after tissue development, is placeable onto exposed bone (5) in the region of a defective articular surface, **characterized in that** the carrier (1), on the side intended for engagement with the bone (5), comprises means for furthering osseous integration (2), and **in that** at least one absorbable sealing layer (3, 4) in the form of a self-supporting film, a membrane and/or a sealing compound is provided to protect against the entry of synovial fluid into the area of contact between the articular surface replacement device (1, 2) and the bone (5).

2. Articular surface replacement device according to any one of the preceding claims, **characterized in that** the porous carrier (1) consists of absorbable material and preferably of at least one absorbable synthetic polymer based on the polyesters of α-hydroxycarboxylic acids.

3. Articular surface replacement device according to any one of the preceding claims, **characterized in that** the porous carrier (1) is formed of a foam, sponge, sintered material or a textile structure, especially a spunbonded web or a staple fibre web.

4. Articular surface replacement device according to any one of the preceding claims, **characterized in that** the integration mediator (2) is firmly bound to the porous carrier (1).

5. Articular surface replacement device according to any one of the preceding claims, **characterized in that** the integration mediator (2) is in finely divided form, the average particle size being preferably between 10 and 500 µm, in particular 50 to 100 µm.

6. Articular surface replacement device according to any one of the preceding claims, **characterized in that** the integration mediator (2) consists of a coherent high-porosity plate.

7. Articular surface replacement device according to any one of the preceding claims, **characterized in that** the integration mediator (2) is present in an amount covering 30% to 100% and preferably 95% to 100% of the surface of the side facing the bone (5).

8. Articular surface replacement device according to any one of the preceding claims, **characterized in that** the integration mediator (2) is formed by, and preferably consists of, an inorganic bone matrix substance and in particular consists of ceramic or amorphous hydroxyapatites, tricalcium phosphates, mixed calcium phosphates and/or calcium carbonate.

9. Articular surface replacement device according to any one of the preceding claims, **characterized in that** the integration mediator (2) is of biological origin and in particular consists of bone subjected to high temperature treatment.

10. Articular surface replacement device according to any one of the preceding claims, **characterized in that** the integration mediator (2) has a porous structure, the porosity preferably being in the range from 30% to 80% by volume and the pore size preferably being in the range from 10 to 150 µm.

11. Articular surface replacement device according to any one of the preceding claims, **characterized in that** the at least one sealing layer (3, 4) protects the area of contact between the implant (1, 2) and defect boundaries on the cartilage (6) and between the integration mediator (2) and the bone (5) in the implanted state against entry of synovial fluid.

12. Articular surface replacement device according to any one of the preceding claims, **characterized in that** the three-dimensional porous carrier (1) is provided, on the side of the integration mediator (2), with an uninterrupted layer (3), in particular with a preferably absorbable self-supporting film or membrane in which the particles of the mediator are anchored.

13. Articular surface replacement device according to any one of the preceding claims, **characterized in that** it is assigned a preferably absorbable covering film or membrane (4) intended to cover the upper side of the carrier (1), remote from the integration mediator (2), and in particular the bondline (7) between the implant and the natural articular surface.

14. Articular surface replacement device according to any one of the preceding claims, **characterized in that** it is assigned an absorbable sealing compound which, introduced between the implant and the implant bed, or applied atop the surficial dividing line from implant to articular surface, prevents penetration by macromolecular constituents of synovial fluid.

15. Articular surface replacement device according to any one of the preceding claims, **characterized in that** the porous carrier (1) and/or the integration mediator (2) have a curved three-dimensional shape conformed to the natural shape of the joint, that side of the integration mediator (2) which faces the bone (5) preferably being planar.

## Revendications

1. Substitut biohybride pour la surface d'une articulation, qui présente la forme d'un support (1) poreux tridimensionnel dans lequel on peut cultiver des cellules de cartilage in vitro et/ou in vivo pour former un pansement cellulaire tridimensionnel et qui, après la croissance cellulaire et éventuellement après un développent tissulaire, peut être placé sur l'os (5) dénudé dans la zone d'une surface défectueuse d'une articulation, **caractérisé en ce que** sur son côté destiné à être posé sur l'os (5), le support (1) présente des moyens (2) qui favorisent l'intégration osseuse et **en ce qu'**au moins une couche résorbable de recouvrement (3, 4) qui présente la forme d'un film, d'une membrane et/ou d'une pâte d'étanchéité est prévue afin d'empêcher l'entrée de liquide synovial dans la surface de contact entre le substitut (1, 2) pour la surface d'une articulation et l'os (5).

2. Substitut pour la surface d'une articulation selon l'une des revendications précédentes, **caractérisé en ce que** le support poreux (1) est constitué d'un matériau résorbable et de préférence d'au moins un polymère synthétique résorbable à base d'un polyester d'acide α-hydroxycarboxylique.

3. Substitut pour la surface d'une articulation selon l'une des revendications précédentes, **caractérisé en ce que** le support poreux (1) est formé à partir d'une mousse, d'une éponge, d'un matériau fritté ou d'une structure textile et en particulier d'un non-tissé ou d'un feutre de fibres discontinues.

4. Substitut pour la surface d'une articulation selon l'une des revendications précédentes, **caractérisé en ce que** l'agent d'intégration (2) est relié solidairement au support poreux (1).

5. Substitut pour la surface d'une articulation selon l'une des revendications précédentes, **caractérisé en ce que** l'agent d'intégration (2) se présente sous forme finement divisée, la granulométrie moyenne étant de préférence comprise entre 10 et 500 µm et en particulier entre 50 et 100 µm.

6. Substitut pour la surface d'une articulation selon l'une des revendications précédentes, **caractérisé en ce que** l'agent d'intégration (2) est constitué d'une plaque continue et très poreuse.

7. Substitut pour la surface d'une articulation selon l'une des revendications précédentes, **caractérisé en ce que** l'agent d'intégration (2) est présent en une quantité qui recouvre entre 30 % et 100 % et de préférence entre 95 % et 100 % de la surface orientée vers l'os (5).

8. Substitut pour la surface d'une articulation selon l'une des revendications précédentes, **caractérisé en ce que** l'agent d'intégration (2) est formé à partir d'une substance minérale osseuse de base, de préférence d'une telle substance et en particulier de céramiques ou d' hydroxyapatites amorphes, de triphosphate de calcium, de phosphates mixtes de calcium et/ou de carbonate de calcium.

9. Substitut pour la surface d'une articulation selon l'une des revendications précédentes, **caractérisé en ce que** l'agent d'intégration (2) est d'origine biologique et est en particulier constitué d'os traité à haute température.

10. Substitut pour la surface d'une articulation selon l'une des revendications précédentes, **caractérisé en ce que** l'agent d'intégration (2) présente une structure poreuse, la porosité étant de préférence comprise entre 30 et 80 % en volume et la taille des pores étant de préférence comprise entre 10 et 150 µm.

11. Substitut pour la surface d'une articulation selon l'une des revendications précédentes, **caractérisé en ce que**, lorsque que l'implantation a été réalisée, l'au moins une couche de recouvrement (3, 4) protège de l'entrée de liquide synovial la surface de contact entre l'implant (1, 2) et les limites de la zone de cartilage (6) endommagée et la surface de contact entre l'agent d'intégration (2) et l'os (5).

12. Substitut pour la surface d'une articulation selon l'une des revendications précédentes, **caractérisé en ce que**, du côté de l'agent d'intégration (2), le support poreux (1) tridimensionnel est dotée d'une couche fermée (3) et est en particulier relié à un film ou à une membrane de préférence résorbables dans lesquels sont ancrées les particules de l'agent.

13. Substitut pour la surface d'une articulation selon l'une des revendications précédentes, **caractérisé en ce qu'**un film ou une membrane (4) de recouvrement de préférence résorbables lui sont associés afin de recouvrir le côté supérieur du support (1), non orienté vers l'agent d'intégration (2), et en particulier pour recouvrir la jonction (7) entre l'implant et la surface native d'articulation.

14. Substitut pour la surface d'une articulation selon l'une des revendications précédentes, **caractérisé en ce qu'**une pâte d'étanchéité résorbable lui est associée et est introduite entre l'implant et le site d'emplacement de l'implant ou est apportée sur la ligne superficielle de séparation entre l'implant et la surface d'articulation pour éviter l'intrusion de composantes macromoléculaires de liquide synovial.

15. Substitut pour la surface d'une articulation selon l'une des revendications précédentes, **caractérisé en ce que** le support poreux (1) et/ou l'agent d'intégration (2) présentent une forme incurvée adaptée à la forme naturelle d'articulation, le côté de l'agent d'intégration (2) tourné vers l'os (5) étant de préférence lisse.
